# DEMANDE DE BREVET EUROPEEN

(11) **EP 4 008 296 A1**
(43) Date de publication de la demande: **08.06.2022**
(21) Numéro de dépôt: 20211055.7
(22) Date de dépôt: 01.12.2020
(51) Int. Cl.: A61F 5/44, A61J 15/00, A61M 39/02, A61M 39/24, A61F 5/445, A61F 5/442, A61M 1/00

(54) **DISPOSITIF COMPORTANT UN RÉSERVOIR DE VOLUME VARIABLE ET PERMETTANT LE PASSAGE D'UNE COMPOSITION NUTRITIVE ET/OU PHARMACEUTIQUE D'UNE STOMIE VERS UN AUTRE ORIFICE NATUREL OU ARTIFICIEL**

(71) Demandeur: CHRU de Lille, 59000 Lille (FR)
(72) Inventeur: LOGIER, Regis Jean-Marc, 59520 Marquette Les Lille (FR); NZAMUSHE LEPAN MABLA, Jean Robert, 1420 Braine L'Alleud (BE); DERROUICHE, Amil, 62820 Libercourt (FR)
(74) Mandataire: Ipside

(57) **Abrégé**

La présente invention concerne un dispositif permettant le passage d'une composition nutritive et/ou pharmaceutique d'une stomie vers un autre orifice naturel ou artificiel du corps d'un patient, ledit dispositif comportant un réservoir de volume variable (1) équipé d'une ouverture de montage (11) apte à être montée de manière étanche autour de ladite stomie et une pompe (7) permettant la circulation du contenu dudit réservoir de ladite stomie vers l'autre orifice, ledit réservoir (1) présentant une dimension longitudinale et comportant une portion de section transversale rétrécie (13), distante de ladite ouverture de montage (11) et s'étendant selon ladite dimension longitudinale dudit réservoir (1). De manière caractéristique, ladite pompe (7) est montée au niveau de l'extrémité distale (131) de ladite portion de section transversale rétrécie (13) dudit réservoir (1).

## Description

### Domaine technique

La présente invention concerne un dispositif médical comportant un réservoir de volume variable et permettant la circulation de bol alimentaire ou tout autre composition nutritive et/ou pharmaceutique entre deux stomies ou entre une stomie et un autre orifice du corps du patient.

### Art antérieur

Le document EP 2 953 593 A1, lequel revendique une date de priorité de 2013, décrit un dispositif externe qui permet de faire circuler le bol alimentaire entre deux stomies. Ce dispositif comporte des moyens de connexion étanche à une stomie amont, des moyens de connexion étanche à une stomie aval et une pompe permettant de faire circuler le bol alimentaire arrivant au niveau de la stomie amont vers la stomie aval. Ce dispositif peut être fixé sur le corps du patient, en particulier sur son abdomen.

Le document WO2019/073365 A1 lequel revendique une priorité de 2017 décrit un dispositif similaire qui diffère du précédent en ce qu'une poche souple permet de faire communiquer les deux stomies. La pompe est une pompe à palettes qui peut être localisée dans la poche, où elle flotte sur le contenu plus ou moins liquide de la poche. Dans un autre mode de réalisation, la pompe est une pompe péristaltique externe à la poche souple. Elle est connectée à la poche souple par deux conduites. La conduite de sortie de la pompe est directement reliée à la stomie aval. Dans un autre mode de réalisation, la pompe est un piston qui comporte une entrée connectée à la poche. La sortie du piston, activé manuellement, est reliée à la stomie aval. Il s'avère que ce type de dispositif est adapté lorsque le contenu de la poche est peu visqueux et homogène. Il est adapté à une composition nutritive artificielle mais pas au bol alimentaire d'un patient alimenté avec des aliments qui ne sont pas tous liquides. De plus, la pompe, du fait de sa disposition, a tendance à se désamorcer ce qui engendre l'arrêt du flux de liquide contenu dans la poche.

Par ailleurs, dans le document précité, la pompe peut contenir une partie interne à la poche et un moteur externe qui est apte à mettre en mouvement la partie interne de manière à aspirer le contenu de la poche. Le moteur doit donc être fixé sur la poche. Le moteur est lourd et n'est pas adapté pour des patients pouvant se tenir debout et pouvant éventuellement marcher car il a tendance à étirer la poche, gênant ainsi l'aspiration du contenu de cette dernière. Le poids du moteur risque également de décoller la poche du corps du patient.

### Problème technique à résoudre

Un but de la présente invention est de proposer un nouveau dispositif qui remédie au moins partiellement aux inconvénients liés au dispositif de l'art antérieur.

### Brève description de l'invention

La présente invention concerne un dispositif permettant le passage d'une composition nutritive et/ou pharmaceutique d'une stomie vers un autre orifice naturel ou artificiel du corps d'un patient, ledit dispositif comportant un réservoir de volume variable équipé d'une ouverture de montage apte à être montée de manière étanche autour de ladite stomie et une pompe permettant la circulation du contenu de la poche de ladite stomie vers l'autre orifice, ledit réservoir présentant une dimension longitudinale et comportant une portion de section transversale rétrécie, distante de ladite ouverture de montage et s'étendant selon ladite dimension longitudinale de dudit réservoir. De manière caractéristique, ladite pompe est montée au niveau de l'extrémité distale de ladite portion de section transversale rétrécie.

Dans la suite de la demande, on désignera par souci de simplicité par les termes « portion rétrécie », la portion de section transversale rétrécie du réservoir de volume variable.

Le réservoir de volume variable peut être, par exemple une poche souple apte à se gonfler pour contenir une quantité variable de composition nutritive et/ou pharmaceutique, et notamment de bol alimentaire ou de chyme. Il peut s'agir également d'un réservoir en accordéon, susceptible de s'étendre selon sa dimension longitudinale sous l'effet du poids de son contenu.

L'extrémité distale (ou extrémité libre) de la portion rétrécie est la portion la plus basse du dispositif (hors pompe) lorsque le dispositif est monté sur l'abdomen du patient. La portion rétrécie est donc toujours remplie du liquide contenu dans le réservoir, en particulier l'extrémité distale de la portion rétrécie. Le fait de monter la pompe au niveau de cette extrémité distale permet d'éviter le désamorçage de la pompe. La pompe est reliée au réservoir et du fait de son poids tend à le maintenir dans une position permettant un bon écoulement du fluide de la stomie dans le réservoir puis de ce dernier vers la pompe. En particulier, dans le cas de patients pouvant se tenir debout et marcher, la pompe va, du fait de la gravité, maintenir le réservoir contre le corps du patient, dans une position verticale, facilitant la sortie du liquide (bol alimentaire ou chyme) hors de la stomie.

Le réservoir ayant un volume variable, il évite les surpressions au niveau de la pompe, laquelle peut ainsi pomper toujours la même quantité de liquide par unité de temps. Il est ainsi possible d'utiliser des pompes de dimensions réduites et de les faire fonctionner avec de faibles pressions, dans le cas des pompes spécifiques qui seront détaillées par la suite.

### Description de l'invention

La portion rétrécie peut s'étendre parallèlement au réservoir ; son extrémité distale peut être à la même distance de l'ouverture de montage que le fond du réservoir ou être plus éloignée que ce dernier.

Selon un mode de réalisation, ladite portion de section transversale rétrécie prolonge ledit réservoir selon ladite dimension longitudinale de ce dernier ce qui assure le remplissage de la portion rétrécie. Un tel réservoir est, de plus, simple à fabriquer.

L'extrémité distale de la portion rétrécie peut être fermée. Dans ce cas, le réservoir contient une partie interne de la pompe, le moteur activant cette partie interne étant lui, externe au réservoir. Le moteur peut par exemple entrainer en mouvement la partie interne de la pompe par des moyens d'entrainement magnétiques disposés à la fois sur le moteur et sur la partie interne de la pompe. Le moteur peut être disposé sur la paroi latérale de l'extrémité distale de la portion rétrécie ou sous l'extrémité distale, laquelle peut former un fond en deux dimensions, de la portion rétrécie.

Avantageusement, la portion de la section transversale rétrécie est ouverte au niveau de son extrémité distale et ladite pompe est montée de manière étanche au niveau de ladite extrémité ouverte. Dans ce cas, le contenu du réservoir tombe dans la pompe, laquelle est externe à la poche et ne peut donc se désamorcer.

Selon un mode de réalisation avantageux combinable aux autres modes de réalisation de l'invention, l'extrémité ouverte de ladite portion de section transversale rétrécie présente une section ovale ou en forme de mandorle. Cette forme particulière permet d'optimiser la section de l'ouverture de l'extrémité de la portion rétrécie. Elle permet également de monter facilement la pompe sur le réservoir.

Selon l'invention, quel que soit le mode de réalisation, la portion rétrécie peut avoir une forme d'entonnoir selon sa dimension longitudinale ; elle se rétrécie donc davantage en direction de son extrémité distale.

Néanmoins, avantageusement, quel que soit le mode de réalisation, ladite portion de section transversale rétrécie présente une section de même surface sur toute sa longueur. Une telle portion rétrécie est facile à fabriquer en même temps que le réservoir lui-même.

Avantageusement, ladite pompe est externe au réservoir, ce qui signifie qu'aucune partie de la pompe n'est située dans le réservoir ou sa portion rétrécie.

La pompe n'est pas limitée selon l'invention. Elle comporte une entrée qui communique avec l'intérieur de la poche et une sortie munie d'un tube de ré-instillation adapté pour être connecté ou inséré dans un autre orifice.

Avantageusement, au moins ladite entrée est munie d'une valve anti-retour disposée en regard de ladite extrémité ouverte de ladite portion de section transversale rétrécie. Le fait de n'équiper que l'entrée de la pompe avec une valve anti-retour permet de simplifier et d'alléger le dispositif ; en particulier, il est ainsi possible d'utiliser les mêmes moyens d'aspiration/injection d'un fluide pour le fonctionnement de la pompe et pour celui du ballonnet formant valve anti-retour. Les inventeurs ont en effet constaté que du fait de la pesanteur, la pompe se remplit naturellement du contenu de la poche, sans action sur la membrane de la pompe pour aspirer le contenu du réservoir vers la pompe.

L'orifice peut être une stomie d'aval rapprochée ou en canon de fusil, un orifice fistuleux d'aval à proximité de la stomie, une stomie d'aval à distance, un orifice fistuleux d'aval à distance, un embout de sonde gastrique ou jéjunale d'aval. S'il s'agit d'une stomie en canon de fusil ou rapprochée, l'ouverture de montage du réservoir est montée autour des deux stomies et le tube de ré instillation traverse l'intérieur du réservoir ; la sortie de la pompe est alors disposée dans le réservoir. Si l'orifice est éloigné de la stomie (ce peut être le cas si cet orifice est une stomie d'aval à distance, un orifice fistuleux d'aval à distance, un embout de sonde gastrique ou jéjunale d'aval), la sortie de la pompe peut rester interne au réservoir et le tube de ré instillation peut alors traverser la paroi du réservoir et de l'intérieur de ce dernier vers l'extérieur. La sortie de la pompe peut également être externe au réservoir et le tube de ré-instillation est alors également externe au réservoir sur toute sa longueur.

Quel que soit le mode de réalisation de l'invention, la pompe est avantageusement du type comportant :
- une entrée munie d'une valve anti-retour disposée en regard de ladite extrémité ouverte de ladite portion de section transversale rétrécie ; et
- une sortie éventuellement équipée d'une valve antiretour et apte à être connectée audit orifice.

Quel que soit le mode de réalisation, la pompe comporte avantageusement une membrane déformable disposée en regard de l'entrée et de la sortie de la pompe et des moyens d'aspiration/injection d'un fluide lesquels sont aptes à déformer la membrane de manière à faire avancer le contenu de la poche ayant traversé l'entrée de ladite pompe vers ladite sortie de ladite pompe. Une face de la membrane est donc en contact avec le contenu du réservoir tandis que l'autre est en contact avec le fluide. Ce fluide peut être de l'air ou un gaz inerte, par exemple. Une telle pompe est facile et peu couteuse à fabriquer. Les moyens précités d'aspiration/injection d'un fluide sont aptes à être montés sur le corps du patient au moyen d'une ceinture ou d'une bretelle, par exemple. Ils peuvent également être logés dans une petite sacoche que le patient porte à l'épaule. Ces moyens d'aspiration/injection ne sont pas jetables et peuvent être réutilisés lors du changement de poche puisque le fluide qu'ils aspirent ou injectent dans la pompe n'est jamais en contact avec le contenu de la pompe. Par rapport à l'art antérieur, la quantité de matière qui doit être éliminée (par incinération dans la plupart des cas) à chaque changement de dispositif est réduite. Le dispositif de l'invention est donc plus économique et plus écologique que celui de l'art antérieur.

Quel que soit le mode de réalisation, la valve anti-retour comporte un ballonnet gonflable qui comporte un col longitudinal lequel présente une section transversale en forme de mandorle. Un tel ballonnet peut être obtenu par fixation d'une membrane ou peau tridimensionnelle dans une cavité. La cavité est reliée à des moyens d'injection/aspiration d'un fluide qui peuvent être les mêmes que ceux utilisés pour la déformation de la membrane de la pompe. Par injection ou aspiration du fluide, la peau formant la valve antiretour se déforme. Lorsque du fluide est injecté dans la cavité, sous l'effet de la pression du fluide, le col du ballonnet se replie sur lui-même et se ferme de manière étanche. Sans pression de fluide dans la cavité, le col du ballonnet reste ouvert et en présente alors une section transversale en forme de mandorle ; il permet ainsi le passage du contenu du réservoir vers la membrane de la pompe, par exemple. Un tel ballonnet réagit à une faible pression du fluide dans la cavité et se ferme et s'ouvre rapidement. Il est ainsi possible de réduire la taille des moyens d'injection/aspiration d'un fluide qui sont utilisés pour la fermeture et l'ouverture de la valve anti-retour. Le dispositif de l'invention est donc plus léger et moins bruyant ce qui est un gain appréciable de confort pour le patient.

Selon un mode de réalisation particulier, combinable à tous les modes de réalisation précités, ladite pompe est montée sur ladite extrémité ouverte de ladite portion de section transversale rétrécie au moyen d'un connecteur, lequel comporte une portion longitudinale creuse solidaire d'un support sur lequel est montée ladite pompe, ladite portion longitudinale creuse est logée et montée de manière étanche dans la portion de section transversale rétrécie dudit réservoir. La portion longitudinale du connecteur est logée dans la portion rétrécie et le support s'étend transversalement à la longueur de la portion rétrécie, au niveau de l'extrémité distale ouverte de la portion rétrécie. Il est ainsi facile de connecter de manière étanche la pompe au réservoir. La paroi de la portion rétrécie est fixée, par exemple par collage ou soudage sur l'extérieur de la portion longitudinale creuse du connecteur, laquelle permet un bon écoulement du contenu du réservoir vers la pompe. La pompe est avantageusement collée sous le support.

Avantageusement, quel que soit le mode de réalisation du connecteur, ladite portion longitudinale creuse présente une section transversale en forme de mandorle. Il est ainsi facile de fixer par soudage ou collage la portion rétrécie du réservoir, en particulier lorsque ce dernier comporte ou est formé de feuille(s) souples (par exemple, une poche) sur la portion longitudinale creuse du connecteur ; la paroi de la portion rétrécie ne forme pas de plis et épouse facilement la paroi externe de la portion longitudinale.

Le réservoir n'est pas limité selon l'invention. Il peut comprendre une portion de volume fixe (une enceinte rigide, par exemple) et une portion souple, éventuellement déformable, laquelle est de volume variable du fait, par exemple de son gonflement possible ou de son extension possible. Le réservoir peut être une poche de stomie existante, par exemple. Cette poche peut être formée d'une seule pièce ou de deux feuilles souples soudées. Avantageusement, ladite poche est formée par soudure de deux feuilles souples, les soudures formant le contour de ladite poche. Il est ainsi facile de former la portion rétrécie lors de la formation de la poche et de souder le connecteur à la portion rétrécie.

Dans tous les cas, le réservoir est étanche et ne laisse pas son contenu s'échapper, à part au niveau de son ou ses ouvertures.

Selon un autre aspect de l'invention, la valve anti-retour comportant un ballonnet gonflable qui comporte un col longitudinal lequel présente une section transversale en forme de mandorle peut être utilisée sans le connecteur et les autres caractéristiques précitées de l'invention. L'invention peut ainsi également concerner un dispositif permettant le passage d'une composition nutritive et/ou pharmaceutique d'une stomie vers un autre orifice naturel ou artificiel du corps d'un patient, ledit dispositif comportant une enceinte éventuellement de volume variable apte à être connectée de manière étanche à une stomie et une pompe permettant la circulation du contenu de ladite enceinte provenant de ladite stomie vers l'autre orifice, ladite pompe comportant une entrée débouchant dans ladite enceinte et une sortie apte à être connectée audit orifice ; ledit dispositif comportant au moins au niveau de ladite entrée un ballonnet gonflable formant valve-antiretour. Ce ballonnet gonflable présente, de manière caractéristique un col longitudinal lequel présente une section transversale en forme de mandorle. Ce dispositif peut également comprendre un réservoir de volume variable, notamment une poche, tel que décrit dans la présente demande. La sortie de la pompe peut également être équipée du même ballonnet précité que l'entrée. Ce dispositif peut également, selon une variante, comporter le connecteur décrit ci-dessus. La pompe n'est pas limitée et peut correspondre à l'un quelconque des modes de réalisation décrits dans la présente demande. Elle peut ainsi comprendre une membrane disposée en regard de ladite entrée et de ladite sortie et séparant une enceinte en deux compartiments, un premier compartiment qui reçoit le contenu de l'enceinte et un deuxième compartiment opposé qui est séparé dudit premier compartiment par ladite membrane. Cette pompe comporte en outre des moyens d'aspiration/d'injection d'un fluide apte à injecter/aspirer un fluide dans ledit deuxième compartiment de manière à déformer ladite membrane pour faire sortir le contenu dudit premier compartiment vers la sortie de la pompe. Le dispositif comporte en outre des moyens d'injection/aspiration d'un fluide dans la ou les valves antiretours.

Quel que soit le mode de réalisation et/ou l'aspect de l'invention, le ballonnet gonflable précité comporte une peau tridimensionnelle qui comporte un col dont la section transversale est en forme de mandorle et une enceinte étanche comportant deux cavités opposées reliées de manière étanche par le col de ladite peau ; l'enceinte comporte en outre un passage de fluide par lequel elle est connectée de manière étanche à des moyens d'aspiration/injection de fluide qui permettent la déformation élastique de la peau en sorte de fermer ou d'ouvrir le col formé par cette dernière.

Quel que soit l'aspect de l'invention ou son mode de réalisation, le dispositif comporte des moyens de détection de la présence de la composition nutritive et/ou pharmaceutique dans l'enceinte ou le réservoir de volume variable ; ces moyens de détection sont adaptés pour mesurer un paramètre variable en fonction de la présence ou non de composition nutritive et/ou pharmaceutique dans l'enceinte ou le réservoir de volume variable et pour actionner la pompe lorsque la valeur dudit paramètre correspond à la présence de composition nutritive et/ou pharmaceutique. Le dispositif comporte, en outre, avantageusement, quel que soit le mode de réalisation ou l'aspect de l'invention, des moyens de déclenchement desdits moyens de détection qui actionnent lesdites moyens de détection au bout d'une durée test choisie par l'utilisateur ; et des moyens de réglage de ladite durée test.

Les moyens de détection précités comportent au moins un capteur choisi parmi les capteurs capacitifs, les capteurs optiques, les capteurs de température et les capteurs de pression.

Les moyens de détection et lesdits moyens de déclenchement de ces derniers rendent le dispositif de l'invention totalement automatique. Même si le patient est, par exemple, dans le coma, le dispositif de l'invention se déclenche sans intervention du personnel soignant. Contrairement au dispositif de l'art antérieur qui nécessite l'intervention soit du patient soit du personnel soignant pour le montage du moteur de la pompe sur la poche, le dispositif de l'invention ne nécessite aucune intervention. En particulier, lorsque la pompe est externe au dispositif et telle que définie ci-dessus (pompe à membrane), le patient n'a pas à s'occuper du fonctionnement du dispositif de l'invention lequel injecte petit à petit et donc sans douleur le contenu de la poche dans l'orifice.

### Définitions

Le terme mandorle désigne une forme géométrique définie par l'intersection de deux cercles de même rayon.

Le terme composition nutritive désigne toute composition contenant des nutriments ; il peut s'agir d'une composition artificielle ou du bol alimentaire provenant de l'estomac du patient ou du chyme.

### Figures

La présente invention, ses caractéristiques et les divers avantages qu'elle procure apparaitront mieux à la lecture de la description qui suit d'un mode de réalisation particulier de l'invention qui fait référence aux dessins annexés, présentés à titre d'exemples illustratifs et non limitatifs sur lesquels :
- La Fig. 1 représente une vue schématique éclatée de face d'un mode de réalisation particulier de l'invention adapté pour une circulation entre deux stomies rapprochées ;
- La Fig. 2 représente une vue schématique en coupe longitudinale du mode de réalisation de la Fig. 1 ;
- La Fig. 3 représente une vue en perspective d'un mode de réalisation particulier du connecteur;
- La Fig. 4a représente une vue en perspective d'un mode de réalisation particulier de la membrane formant le ballonnet anti-retour de la pompe;
- la Fig. 4b représente le ballonnet formé dans la cavité représentée sur la Fig. 2; et
- la Fig. 4c représente les étapes de fermeture du ballonnet représenté sur la figure 4b.

### Exemples

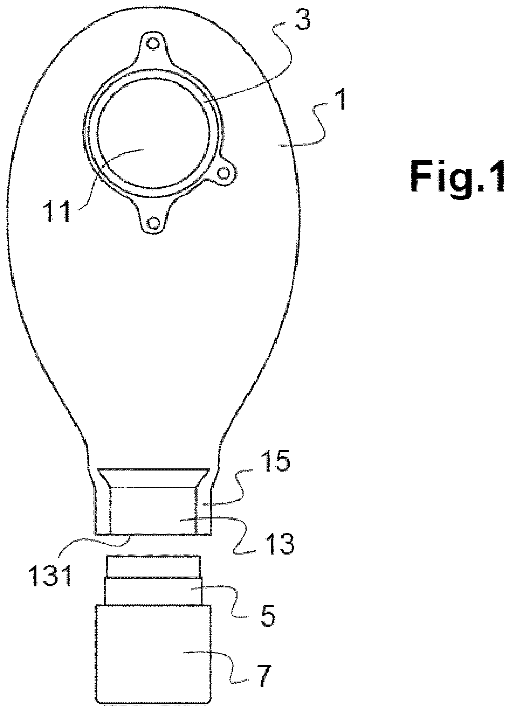

Dans le mode de réalisation décrit, le réservoir de volume variable est une poche 1. En référence à la Fig. 1, le dispositif de l'invention comporte une poche souple 1. La poche 1 est formée par soudage de deux feuilles souples ; les soudures forment les bords de la poche 1. Cette poche souple comporte une ouverture de montage 11 entourée d'une pièce de montage 3. La pièce de montage 3 est fixée de manière étanche au niveau de l'ouverture 11 et permet la fixation de la poche sur un support collé sur l'abdomen du patient, autour d'une stomie. Ce type de fixation est déjà connu dans le domaine des poches adaptées pour récupérer le bol alimentaire ou le chyme sortant d'une stomie. La poche 1 comporte dans sa partie basse une portion de section transversale rétrécie 13. Dans le mode de réalisation représenté, la portion rétrécie 13 prolonge la poche 1 selon sa longueur et forme la partie distale de la poche. La portion rétrécie 13 est délimitée par des soudures 15. L'extrémité distale de la portion rétrécie 131 est ouverte. Le connecteur 5 est enfoncé dans la portion rétrécie 131 et fixé à cette dernière de manière étanche. La pompe 7 est fixée de manière étanche sur le connecteur 5, à l'opposé de la poche 1.

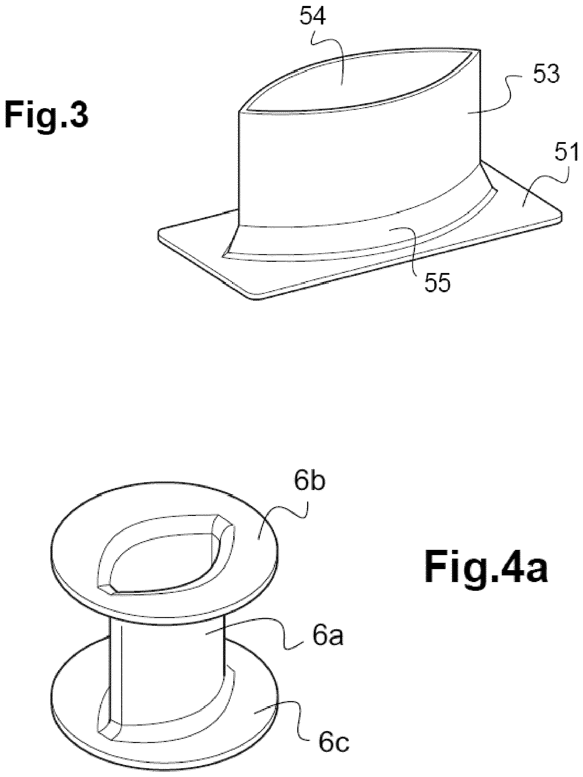

Sur la Fig. 1, la paroi latérale de la portion rétrécie 131 est éloignée de la portion longitudinale creuse 53 du connecteur 5 par souci de simplification. En réalité, la paroi latérale de la portion rétrécie 13 est fixée sur la paroi externe de la portion longitudinale 53 (voir Fig. 3) du connecteur 5.

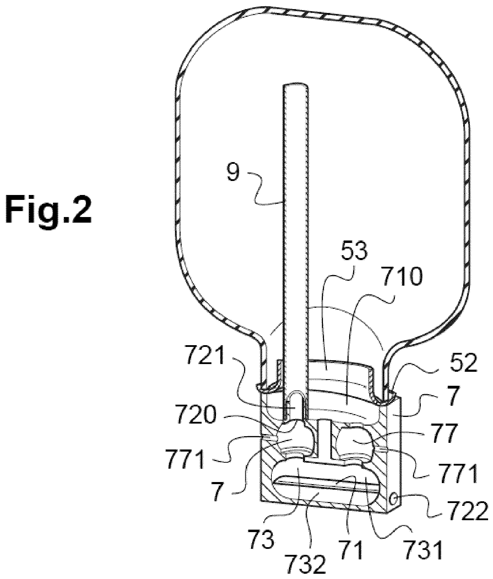

En référence à la Fig. 2, la pompe 7 comporte une membrane déformable 71 qui divise la cavité de circulation 73 en deux compartiments étanches : un premier compartiment 731 en regard de l'entrée 710 et de la sortie 720 et un deuxième compartiment 732 séparé du premier compartiment 731 par la membrane déformable 71. La membrane déformable 71 s'étend sous l'entrée 710 et sous la sortie 720 de la pompe 7. Un passage de fluide 722 est ménagé dans la paroi de la pompe 7, au niveau de la cavité de circulation 73 ; ce passage de fluide 722 débouche dans le deuxième compartiment 732. Ce passage 722 est relié à des moyens d'injection/aspiration d'un fluide qui ne sont pas représentés sur la Fig. 2. L'entrée 710 et la sortie 720 de la pompe 7 communiquent chacune avec une cavité 77 dans laquelle est logé un ballonnet servant de valve anti-retour. Par souci de simplification, les ballonnets ne sont pas représentés sur la Fig. 2. Chaque cavité 77 comporte un passage 771 qui permet à un fluide mis en mouvement par des moyens d'injection/d'aspiration d'un fluide (non représentés) de faire gonfler ou au contraire de vider le ballonnet contenu dans la cavité 77. Une buse de sortie 721 est montée sur la cavité au niveau de la sortie 720. Cette buse de sortie 721 est insérée de manière étanche dans le tube de ré-instillation 9.

Les moyens de détection de la présence de composition nutritive et/ou pharmaceutique dans la poche 1 ne sont pas représentés. Il en est de même des moyens d'aspiration/injection d'un fluide qui sont connectés aux ballonnets formant valve anti-retour et de ceux connectés au compartiment 732 de la pompe 7, lesquels peuvent être les mêmes.

Selon une variante, seule la cavité 77 en regard de la sortie 720 est équipée d'un ballonnet formant valve anti-retour. Du fait de la gravité, la valve anti-retour au niveau de l'entrée n'est pas nécessaire. Le dispositif est ainsi simplifié et allégé. Il est alors possible d'utiliser les mêmes moyens d'injection/aspiration d'un fluide pour le fonctionnement de la pompe 7 et pour l'ouverture et la fermeture du ballonnet formant valve anti-retour au niveau de l'entrée de la pompe 7.

En référence à la Fig. 3, un mode de réalisation particulier du connecteur va maintenant être décrit. Les parties en commun avec le connecteur 5 représenté sur les Fig. 1 et 2 sont référencées à l'identique. Le connecteur 5 comporte une portion longitudinale creuse 53 qui s'étend depuis un socle 51. La portion longitudinale creuse 53 est verticale tandis que le support/socle 51 s'étend dans un plan horizontal. La portion longitudinale creuse 53 présente une section transversale 54 en forme de mandorle qui s'évase vers le support 51. Le support 51 est ouvert en regard de la portion longitudinale creuse 53. La paroi interne de la portion longitudinale creuse 53 a une section transversale constante sur toute sa hauteur. Sur l'extérieur du connecteur 5 une portion de renfort 55 permet de bien solidariser la portion longitudinale creuse 53 au support 51. Le connecteur 5 peut être, par exemple, fabriqué par moulage.

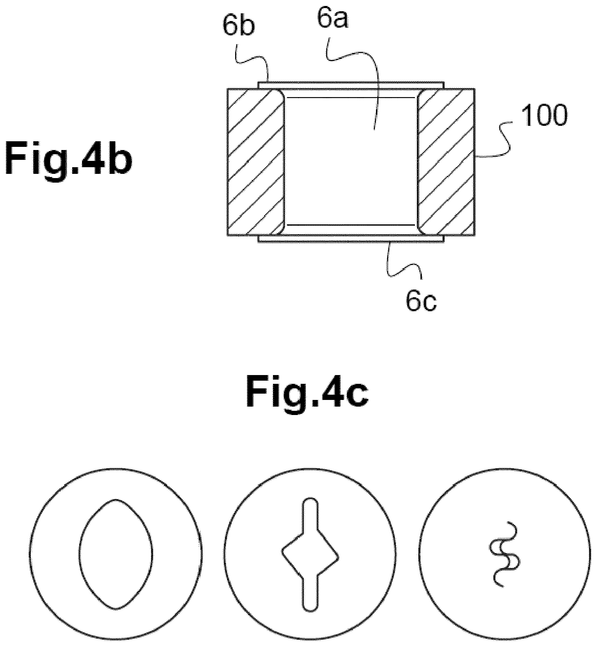

En référence aux figures 4a à 4c, un mode de réalisation particulier de la valve anti-retour ou ballonnet va maintenant être décrit. Une membrane ou peau 6 élastiquement déformable et ayant une forme tridimensionnelle est montée et fixée dans une enceinte 100 (visible sur la Fig. 4b). La peau 6 a une forme de bobine. Elle comporte un corps 6a longitudinal et deux lèvres 6b et 6c qui sont solidaires des extrémités du corps 6a.

Dans l'exemple représenté, les deux lèvres ont un bord circulaire et s'étendent dans un plan perpendiculaire à la longueur du corps 6a. Le corps 6a de la peau présente une section transversale en forme de mandorle. Les angles qui correspondent à l'intersection des deux cercles ne sont pas vifs mais comportent en fait un petit méplat ou un arrondi afin de permettre le démoulage de la peau. En référence à la Fig. 4b, l'enceinte 100 est une petite boîte qui présente deux ouvertures circulaires sur deux de ces faces opposées. La peau 6 est insérée dans l'enceinte 100 et ses lèvres 6b et 6c sont, par exemple, collées de manière étanche sur la portion de l'enceinte 100 entourant les ouvertures circulaires. On obtient ainsi une enceinte étanche à l'air, par exemple et un ballonnet dont le col présente une section transversale en forme de mandorle.

La Fig. 4c montre la fermeture du ballonnet formé par la peau élastiquement déformable 6 lorsqu'un fluide pouvant être de l'air, est insufflé dans l'enceinte 100. L'air sous pression vient presser la paroi externe du corps 6a, ce qui déforme ce dernier. La déformation n'est pas régulière et le corps 6a se déforme par vagues jusqu'à ce que les deux parois en regards s'accolent l'une contre l'autre en une série de courbes. La dernière figure de la Fig. 4c représente le ballonnet juste avant sa fermeture totale. Le ballonnet fermé s'avère être plus étanche qu'un ballonnet torique. La surface de contact entre les deux lèvres du ballonnet est en effet plus importante du fait des vagues. De plus, le ballonnet de la Fig. 4 se ferme avec une pression moindre par rapport à un ballonnet torique de même dimension ce qui permet de réduite la taille et puissance des moyens d'injection/aspiration d'un fluide, lesquels peuvent de plus être bruyants.

Un exemple de fonctionnement du dispositif de l'invention va maintenant être décrit en référence aux Fig. 1 à 4.

La poche 1 peut être vide ou contenir une composition/solution nutritive et/ou pharmaceutique. La poche 1 est montée autour d'une stomie. Le tube de ré-instillation 9 est inséré dans un autre orifice. Si le second orifice est une stomie rapprochée de la première, la poche 1 est montée autour des deux stomies et le tube de ré-instillation 9 est inséré dans la deuxième stomie ; il est situé dans la poche 1. Si l'orifice est un orifice naturel du corps du patient, par exemple une narine, le tube de ré-instillation peut être au moins partiellement externe à la poche 1.

Lorsque les moyens d'injection/aspiration d'un fluide - lesquels sont fixés sur son corps ou facilement accessibles s'ils sont logés dans une sacoche, ceinture ou autre- sont activés par les moyens de détection de la présence de composition nutritive et/ou pharmaceutique dans la poche 1, le ballonnet logé dans la cavité 77 se vide de son air à travers un tube passant dans le passage 771. Le ballonnet s'ouvre ainsi et laisse passer le contenu de la poche 1 dans le premier compartiment 731 de la cavité 73 de la pompe 7. De l'air ou un autre fluide est ensuite injecté dans le deuxième compartiment 732 à travers le passage de fluide 771. La membrane 71 se déforme et fait passer le contenu de la poche vers le tube de ré instillation 9, à traverse un second ballonnet disposé en regard de la sortie 720 de la pompe. Ce second ballonnet est relié à des moyens d'injection/aspiration d'un fluide et est ouvert pour laisser passer la composition vers le tube de ré instillation 9. Le contenu de la poche 1 (composition) passe ainsi dans la portion de l'intestin qui débouche au niveau de la deuxième stomie.

Selon une variante non représentée, seule la cavité 77 en regard de l'entrée 710 est équipée d'une valve anti-retour. La valve se ferme lorsque la pompe 7 pousse la composition vers la deuxième stomie ou l'autre orifice.

Lorsque le patient ne possède plus qu'une portion d'intestin débouchant au niveau d'une stomie (la deuxième portion débouchant au niveau de l'anus ayant été retirée), il est possible d'insérer le tube de ré instillation dans une narine du patient ou dans une stomie pratiquée dans l'estomac du patient. En faisant recirculer plusieurs fois le contenu de la poche dans la portion de l'intestin encore présente chez le patient, on parvient à faire absorber les nutriments et/ou les médicaments contenus dans la poche 1.

Les moyens d'injection/aspiration d'un fluide permettent de commander la pompe 7 et les ballonnets 6 ou le ballonnet anti-retour 6 de cette dernière. Ainsi le ballonnet 6 situé en regard de l'entrée 710 de la pompe 7 est fermé lorsque la pompe 7 injecte la composition qu'elle contient dans le tube de ré instillation 9.

## Revendications

1. Dispositif permettant le passage d'une composition nutritive et/ou pharmaceutique d'une stomie vers un autre orifice naturel ou artificiel du corps d'un patient, ledit dispositif comportant un réservoir de volume variable (1) équipé d'une ouverture de montage (11) apte à être montée de manière étanche autour de ladite stomie et une pompe (7) permettant la circulation du contenu dudit réservoir de ladite stomie vers l'autre orifice, ledit réservoir (1) présentant une dimension longitudinale et comportant une portion de section transversale rétrécie (13), distante de ladite ouverture de montage (11) et s'étendant selon ladite dimension longitudinale dudit réservoir (1), **caractérisé en ce que** ladite pompe (7) est montée au niveau de l'extrémité distale (131) de ladite portion de section transversale rétrécie (13) dudit réservoir (1).

2. Dispositif selon la revendication 1, **caractérisé en ce que** ladite portion de section transversale rétrécie (13) prolonge ledit réservoir (1) selon ladite dimension longitudinale dudit réservoir (1).

3. Dispositif selon la revendication 1 ou 2, **caractérisé en ce que** ladite portion de la section transversale rétrécie (13) est ouverte au niveau de son extrémité distale (131) et **en ce que** ladite pompe (7) est montée de manière étanche au niveau de ladite extrémité ouverte (131).

4. Dispositif selon l'une quelconque des revendications précédentes, **caractérisé en ce que** ladite extrémité ouverte (131) de ladite portion de section transversale rétrécie (13) présente une section ovale ou en forme de mandorle.

5. Dispositif selon l'une quelconque des revendications précédentes, **caractérisé en ce que** ladite portion de section transversale rétrécie (13) présente une section de même surface sur toute sa longueur.

6. Dispositif selon l'une quelconque des revendications précédentes, **caractérisé en ce que** ladite pompe (7) comporte une entrée (710) qui communique avec l'intérieur dudit réservoir (1) et une sortie (720) munie d'un tube de ré-instillation (9), adapté pour être connecté ou inséré dans un autre orifice.

7. Dispositif selon la revendication 6, **caractérisé en ce qu'**au moins ladite entrée (710) est munie d'une valve anti-retour disposée en regard de ladite extrémité ouverte (131) de ladite portion de section transversale rétrécie (13).

8. Dispositif selon la revendication 6, **caractérisé en ce que** ladite valve anti-retour comporte un ballonnet gonflable (6), lequel comporte un col longitudinal (6a) qui présente une section transversale en forme de mandorle.

9. Dispositif selon l'une quelconque des revendications 3 à 8, **caractérisé en ce que** ladite pompe (7) est montée sur ladite extrémité ouverte (131) de ladite portion de section transversale rétrécie (13) au moyen d'un connecteur (5), lequel comporte une portion longitudinale creuse (53) solidaire d'un support (51) sur lequel est montée ladite pompe (7) et **en ce que** ladite portion longitudinale creuse (53) est logée et montée de manière étanche dans la portion de section transversale rétrécie (13) dudit réservoir (1).

10. Dispositif selon la revendication 9, **caractérisé en ce que** ladite portion longitudinale creuse (53) dudit connecteur (5) présente une section transversale en forme de mandorle.
